# EUROPÄISCHE PATENTSCHRIFT

(11) **EP 0 935 461 B1**
(45) Veröffentlichungstag und Bekanntmachung des Hinweises auf die Patenterteilung: **30.07.2003**
(21) Anmeldenummer: 97945778.5
(22) Anmeldetag: 23.10.1997
(51) Int. Cl.: A61K 31/13, A61P 31/12

(54) **ADAMANTANAMINENDERIVATE ZUR BEKÄMPFUNG VON VIREN DER FAMILIE BORNAVIRIDAE**
ADAMANTANE AMINE DERIVATIVES FOR THE TREATMENT OF VIRUSES OF THE BORNAVIRIDAE FAMILY
DERIVEES D'AMINES D'ADAMANTANE POUR LUTTER CONTRE LES VIRUSES DE LA FAMILLE BORNAVIRIDAE

(30) Priorität: 30.10.1996 DE 19644998
(43) Veröffentlichungstag der Anmeldung: 18.08.1999
(73) Patentinhaber: Ludwig, Hanns, 14163 Berlin (DE); Dietrich, Detlef, 30196 Isernhagen (DE); Emrich, Hinderk M., 30659 Hannover (DE)
(72) Erfinder: LUDWIG, Hanns, D-14163 Berlin (DE); DIETRICH, Detlef, D-30196 Isernhagen (DE); EMRICH, Hinderk, M., D-30659 Hannover (DE); BODE, Liv, D-14163 Berlin (DE)
(74) Vertreter: Gramm, Werner, Prof. Dipl.-Ing.
(86) Internationale Anmeldenummer: DE9702455
(87) Internationale Veröffentlichungsnummer: WO98018457

(56) Entgegenhaltungen:
- WO-A-92/17168
- DE-A- 3 921 062
- US-A- 4 174 407
- L. BODE ET AL: "Amantadine and human Borna disease virus in vitro and in vivo in an infected patient with bipolar depression" THE LANCET, Bd. 349, 18.Januar 1997, Seiten 178-179, XP002056298
- DATABASE MEDLINE US NATIONAL LIBRARY OF MEDICINE (NLM), BETHESDA, MD, US AN: 97233300, L. BODE ET AL: "First isolates of infectious human Borna disease virus from patients with mood disorders" XP002056299 in der Anmeldung erwähnt
- BRIESE T ET AL: "GENOMIC ORGANIZATION OF BORNA DISEASE VIRUS" PROCEEDINGS OF THE NATIONAL ACADEMY OF SCIENCES OF USA, Bd. 91, 1.Mai 1994, Seiten 4362-4366, XP002003340 in der Anmeldung erwähnt
- VANDEWOUDE S ET AL: "A BORNA VIRUS CDNA ENCODING A PROTEIN RECOGNIZED BY ANTIBODIES IN HUMANS WITH BEHAVIORAL DISEASES" SCIENCE, Bd. 250, Nr. 4985, 30.Oktober 1990, Seiten 1278-1281, XP000572615

## Beschreibung

Die Erfindung betrifft die Verwendung von Adamantanaminen (Amantadinen) zur Herstellung eines Medikamentes als Mittel zur Bekämpfung von Viren der Familie Borna viridae, insbesondere von Borna Disease Virus und zur Behandlung und Prophylaxe von mit Borna Disease Virus-Infektionen verbundenen Störungen bei Mensch und Tier, wobei ds Amantadin ein 1-Amino-substituiertes, ein 1-Alkylamino-substituiertes oder 1-Amin-alkylsubstituiertes Adamantan, bzw. ein pharmazeutisch vertretbares Salz hiervon ist.

Affekterkrankungen im allgemeinen und Despressionen verschiedener Ausprägungen im besonderen zählen heute wenigstens in den Industrieländern zu den am weitesten verbreiteten Krankheitsbildern. Man schätzt, daß beispielsweise in Deutschland ca. 5 bis womöglich 10 % der Bevölkerung zumindest zeitweise unter depressiven Krankheitszuständen zu leiden haben. Hierbei geht es nicht um leichte psychische Verstimmungen, sondern um Krankheiten, die die Betroffenen stark belasten und nicht nur bei längerem Verlauf arbeitsunfähig machen können. Die Suizidrate für endogen depressiv Kranke liegt derzeit bei 20 %, wenn keine ärztliche Hilfe in Anspruch genommen wird. Oft sind daher lange stationäre Behandlungen unumgänglich, was wiederum Probleme für den Patienten wie auch für die Allgemeinheit mit sich bringt. Bei langer stationärer Behandlung besteht für den Kranken die Gefahr der Hospitalisierung. Von Seiten der Gesellschaft ist es notwendig, genügend Therapieplätze zur Verfügung zu stellen, einschließlich geeigneter Kliniken und speziell geschulten Personals. Dies alles ist auch mit hohen Kosten für die Allgemeinheit verbunden. Wie man heute weiß, können nicht nur Menschen sondern in analoger Weise auch Tiere an affektiven Störungen leiden. Bekannt ist dies zum Beispiel von Pferden, bei denen sich das Krankheitsbild als apathische Phase äußert, die mit Schläfrigkeit, Energielosigkeit und häufig mit Störung der Bewegungskoordination einhergeht. Diese Pferdeerkrankung ist mit einer aktivierten Borna-Disease-Virusinfektion assoziiert. Im Extremfall muß das kranke Tier eingeschläfert werden, um seinem Leiden ein Ende zu bereiten.

Depressionen werden auch medikamentös behandelt. Auf Basis verschiedener Theorien wird dabei versucht, Einfluß auf das Nervensystem zu nehmen und die depressiven Symptome zu bessern. Verschiedene Antidepressiva können in unterschiedlichem Ausmaß stimmungsaufhellend, psychomotorisch aktivierend, dämpfend, oder Angst-reduzierend usw. wirken. Der Wirkungsmechanismus ist bei den meisten Mitteln nur unzureichend geklärt.

In Gebrauch sind beispielsweise trizyklische und tetrazyklische Mittel. Nach anderen therapeutischen Ansätzen werden selektive Serotonin-Wiederaufnahme-Hemmer (SSRI = specific serotonin reuptake inhibitors), Monoaminoxidase-Hemmer oder Noradrenalin-Wiederaufnahme-Hemmer gegeben. Viele Mittel haben relativ starke Nebenwirkungen, wie Krampfgefahr, Tremor, Übelkeit und Erbrechen, Kopfschmerzen, Angstzustände, schwere Leber- und Nierenstörungen, Anämien usw. Bei einer Reihe von Patienten ist die antidepressive Wirkung zudem eingeschränkt oder subjektiv nicht vorhanden.

Es besteht daher ein starkes Bedürfnis für eine wirksamere und nebenwirkungsärmere medikamentöse Behandlung von Affektstörungen verschiedener Genese.

Vor kurzem konnten von den Anmeldern in Blutproben psychisch kranker Patienten während akuter depressiver Phasen Virusproteine und genetisches Material des Borna Disease Virus (BDV) gefunden werden, eines eingehüllten, nicht-segmentierten Einzelstrang-RNA-Virus negativer Polarität, von dem bekannt ist, daß es bei Tieren psychische, zum Teil depressionsartige Symptome auslöst. Das infektiöse humane BDV konnte aus dem Blut von Patienten mit unterschiedlichen Ausprägungsformen von Affektkrankheiten isoliert werden (L. Bode in Curr Topics Microbiol Immun 1995, 190, 103 - 130; L. Bode, W. Zimmermann, R. Ferszt, F. Steinbach, H. Ludwig, Nature Med. 1995, 1, 232 - 236; L. Bode, R. Dürrwald, F. A. Rantam, R. Ferszt, H. Ludwig, Mol. Psychiatry 1996 1(3): 200 - 212).

Es kann vermutet werden, daß eine BDV-Infektion, neben anderen Faktoren wie möglicherweise einer genetischen Veranlagung, einen Faktor bei der Auslösung von Affekterkrankungen, die zum Beispiel durch Veränderungen im Botenstoffbereich des limbischen Systems gekennzeichnet sind, und anderer insbesondere cerebraler Störungen darstellen könnte. Das der Erfindung zugrundeliegende Problem besteht darin, daß Depressionen bei Mensch und Tier medikamentös möglichst wirksam ohne wesentlich beeinträchtigende Nebenwirkungen behandelt werden sollen. Eine gleichzeitig vorhandene BDV-Infektion sollte nach Möglichkeit wirksam zurückgedrängt oder beseitigt werden. Ferner sollen allgemein BDV-Infektionen bei Mensch und Tier bekämpft werden.

Zur Lösung dieser Probleme sieht die Erfindung die Verwendung von Adamantanaminen zur Herstellung eines Medikamentes als Mittel zur Bekämpfung von Viren der Familie Borna viridae, insbesondere von Borna Disease Virus, und zur Behandlung und Prophylaxe von mit Borna Disease Virus-Infektionen verbundenen Störungen bei Mensch und Tier vor, wobei das Amantadin ein 1-Amino-substituiertes, ein 1-Alkylamino-substituiertes oder ein 1-Aminalkyl-substituiertes Adamantan, bzw. ein pharmazeutisch vertretbares Salz hiervon ist. Die erfindungsgemäße Verwendung ist angezeigt bei der Prophylaxe und der Bekämpfung von Viren der Familie *Bornaviridae*, speziell Borna Disease Virus (BDV)-Infektionen und zur Behandlung und anderen mit Borna Disease Virus-Infektionen verbundenen Störungen bei Mensch und Tier, inbesondere von Affekterkrankungen. Die erfindungsgemäße Verwendung kann auch angezeigt sein bei der Prophylaxe und Bekämpfung von Infektionen bei Mensch und Tier mit anderen Viren der Ordnung *Mononegavirales.* Ein negativer Antikörperbefund schließt eine BDV-Infektion und damit eine Indikation für die erfindungsgemäße Behandlung nicht aus.
Da der Mechanismus der Adamantanaminwirkung noch nicht aufgeklärt ist, kann noch nicht mit Sicherheit gesagt werden, ob die Wirkung ursächlich oder indirekt ist. 1-Adamantanamine gehören zur Gruppe der Adamantane. Dies sind stabile, farblose, kristalline, trizyklische Verbindungen mit "käfigartiger" Struktur. Adamantan (Tricyclo[3,3,1,1^{3,7}]decan) selbst (MG 136,23; C₁₀H₁₆) (I) gleicht im räumlichen Aufbau dem Diamant, ist wasserunlöslich und schon deshalb pharmazeutisch unbrauchbar.

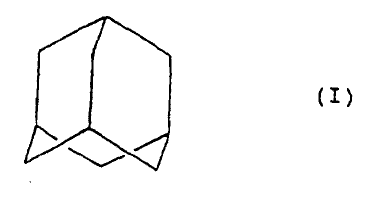

Es gibt allerdings bestimmte, pharmazeutisch verwencbare Adamantanderivate.

Aus der PCT WO 94/28885 sind eine Vielzahl von an verschiedenen Positionen substituierten Adamantanderivaten bekannt - und zwar andere als aminosubstituierte Derivate, insbesondere Alkohole und Ketone. Diesen werden zahlreiche, nicht näher quantifizierte antivirale, antibakterielle, antimykotische und Antitumor-Eigenschaften zugeschrieben. Bestimmte Ketone sollen gegen HIV und ggf. andere Retroviren einsetzbar sein.

Die wasserlöslichen Salze des 1-Adamantanamins oder 1-Aminoadamantans (internationaler Freiname (INN): Amantadin, C₁₀H₁₇N, MG 151,26) Amantadinsulfat (II) und Amantacinhydrochlorid (III) sind seit ca. 30 Jahren als pharmazeutische Wirkstoffe bekannt.

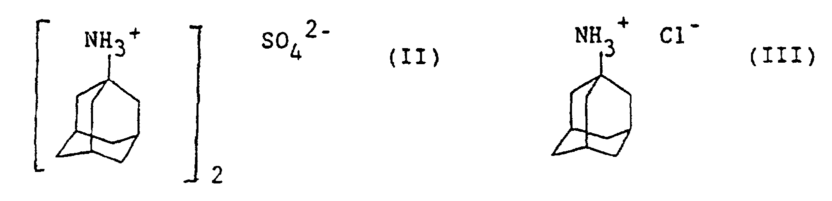

Amantadinsulfat oder -hydrochlorid wird heute noch zur Behandlung von Morbus Parkinson und im weiteren Bereich des Parkinson-Syndroms bei akinetischer Krise, extrapyramidalen Störungen und verminderter Vigilanz verwendet. Die pharmakologische Wirkung beruht dabei, soweit bekannt, u.a. auf einer Erhöhung der Verfügbarkeit von Dopamin an den dopaminergen Synapsen. Der Mechanismus im einzelnen ist noch nicht aufgeklärt. Ausgehend von dieser Wirkung auf das Nervensystem wurde auch bereits die Verwendung zur prophylaktischen Migränetherapie vorgeschlagen (DE 19510189 A1).

Ursprünglich zugelassen wurden wasserlösliche Amantadinderivate für die Prophylaxe und Therapie bei bestimmten Influenza-A-Stämmen. W. L. Davies, R. R. Grunert et. al. (Science 1964, Vol. 144, 862/863) fanden heraus, daß Amantadin-Hydrochlorid die Virusvermehrung bei vier Stämmen von Influenza A und einem C-Typ inhibierte. Dagegen waren schon die nahe verwandten Influenza-B-Stämme, Mumps und eine Vielzahl weiterer RNA- und DNA-Viren nicht sensitiv. Die Virusproduktion konnte bei den A-Stämmen um eine Zehnerpotenz zurückgedrängt, allerdings nicht wirklich unterdrückt werden. Amantadinsulfat und Amantadinhydrochlorid wurden daraufhin zur Prophylaxe von Influenza-A-Infektionen und zur Therapie akuter, durch Influenza-A-Viren hervorgerufener Grippe-Erkrankungen als Arzneimittel international zugelassen.

Die spezifische Aktivität der Amantadine gegen Influenza-A-Viren ist in vitro (Zellkultur) und am Patienten dokumentiert. Der Wirkungsmechanismus beruht auf der Interaktion der Substanz mit einem Influenza-A-spezifischen Membranprotein, M₂, und ist somit für diese Viren spezifisch. Bereits Influenza-B-Viren, die kein M₂-Protein enthalten, sind insensitiv. Durch prophylaktische Gabe werden Häufigkeit und Schweregrad einer durch bestimmte A-Viren hervorgerufenen Virusgrippe gemildert. Wird die Substanz in den ersten 24 - 48 Std. nach dem Auftreten der Grippesymptome gegeben, kann sich die Krankheitsdauer verkürzen (R. Dolin, R. C. Reichman, H. P. Madore, R. Maynard, P. N. Linton, J. Webber-Jones, N. Engl. J. Med. 1982, 307, 580 - 584; W. L. Wingfried, D. Pollack, R. R. Grunert, N. Engl. J. Med. 1969, 281, 579 - 584; A. J. Hay et. al., EMBO J 1985, 4, 3021 - 3024; R. A. Lamb, S. L. Zebedee, C. D. Richardson, Cell 1985, 40, 627 - 633). Die virustatische Wirkung gegen Influenza ist also begrenzt.

Aus der FR 6482M sind als Alternative zu 1-Adamantanaminen bei der Grippe-Therapie auch 2-Adamantanamine mit ähnlicher Wirkung bekannt.

Wegen des Auftretens Amantadin-resistenter Influenza-A-Stämme (F. G. Hayden et al., N. Engl. J. Med. 1989, 321, 1696 - 1702) und der damit verbundenen Gefahr, sowie der Möglichkeit einer Schutzimpfung für Risikopatienten als Alternative ist dieser Indikationsbereich des Medikaments (Grippe) praktisch nicht mehr von Bedeutung.

Überraschenderweise wurde nun gefunden, daß Amantadine hervorragende Wirkung gegen Bornaviren zeigen. Unter Amantadinen werden hier in erster Linie 1-Adamantanamin bzw. seine pharmazeutisch vertretbaren Salze (z. B. Amantadinsulfat, Amantadinhydrochlorid), sowie die 1-Amino-substituierten, 1-Alkylaminosubstituierten oder 1-Aminoalkyl-substituierten Adamantane bzw. deren Salze verstanden (z. B. N-1-Adamantyl-2-[(2-dimethylamino)ethoxy]acetamid = Tromantadin, oder 1-Adamantyl-(1-amino)ethan = Rimantadin, bzw. deren Hydrochloride oder Sulfate).

Die hervorragenden antiviralen Eigenschaften gegenüber BDV sind umso erstaunlicher als eine Chemotherapie von Viruserkrankungen in der Literatur als generell sehr schwierig angesehen wird, da eine kausale Behandlung wegen der "Benutzung" des Stoffwechsels der Wirtszelle immer problematisch erscheint (Mutschler, "Arzneimittelwirkungen" WVG Stuttgart, 7. Aufl. 1996, S. 725, 9.2.4.).

Zwar ist es aus der DE 39 21 062 A1 bekannt, daß 1-Amantadin-Hydrochlorid, allerdings im wesentlichen nur in Kombination mit AZT, gegen HIV-Viren durch Hemmung der Virusvermehrung wirksam sein soll - in ähnliche Zielrichtung geht die zytoprotektive Wirkung, die gemäß DE 40 14 672 A1 mit bestimmten 1-Adamantanamin-Derivaten erreicht wird - allerdings handelt es sich hierbei um Wirkungen speziell auf Retroviren, die einen ganz anderen Lebenszyklus und eine ganz andere Vermehrungsweise haben als die Bornaviren.
Das Bornavirus (Borna Disease Virus, BDV) ist ein eingehülltes RNA-Virus von 90 nm Durchmesser, das ein unsegmentiertes, einzelsträngiges Genom negativer Polarität besitzt, welches für fünf virale Gene kodiert (T. Briese, A. Schneemann, A. J. Lewis, et al., Proc. natl. acad. Sci USA 1994, 91, 4362 - 4366). Verwandte Viren sind z. B. Masernvirus und Tollwutvirus. Bornavirus wird aufgrund genetischer Besonderheiten (Vermehrung im Kern der Wirtszelle, nicht im Cytoplasma) als Prototyp einer eigenen Virusfamilie (*Bornaviridae*) innerhalb der Ordnung *Mononegavirales* angesehen.

BDV war zuerst als tierpathogenes Virus bekannt, das episodische Verhaltensstörungen bei Tieren auslösen kann. BDV-Stämme aus Tieren und Menschen sind genetisch sehr ähnlich (>95% Sequenzhomologie!). BDV hat eine besondere Präferenz für Nervenzellen des limbischen Systems im Gehirn, das für Verhaltenssteuerung, Emotionen und Gedächtnisleistungen (mit-) verantwortlich ist (H. Ludwig, L. Bode, G. Gosztony, Progr. Med. Virol. 1988, 35, 107 - 151; Dittrich W. et al., Biol. Psychatry 1989, 26, 818-828).

Vermutlich kommt es durch (die aktivierte) BDV-Infektion primär zu funktionellen Störungen im Bereich der Gehirnbotenstoffe dieses Systems (G. Gosztony, H. Ludwig, Curr. Topics Microbiol. Immun. 1995, 190, 39 - 73). BDV-Infektionen zerstören die Wirtszelle nicht, sie persistieren und sind durch latente und aktivierte Phasen gekennzeichnet. Insbesondere während der Aktivitätsphasen kann es zu Krankheitssymptomen kommen.

Wasserlösliche Abkömmlinge der 1-Adamantanamine hemmen die Infektion mit Borna Disease Virus (in vitro zu 80 %) und verhindern die Virusreplikation in bereits infizierten Zellen. Das Ausmaß dieses Effekts (in den Beispielen näher beschrieben) ist außerordentlich hoch und völlig unerwartet. Bei der Chemotherapie von Viren sind derartig gute virustatische Ergebnisse unbekannt oder sehr selten. Die Infektion wird vermutlich sogar vollständig beseitigt (Virus-clearance), siehe unter, Beispiel 2 und 3, wobei diese viruzide Wirkung völlig überraschend ist.

Ferner wirken die hier beschriebenen und beanspruchten Stoffe (in vivo) als hocheffiziente Therapeutika bei Affekterkrankungen und bei Störungen, die im Zusammenhang mit BDV-Infektionen stehen.

Diese Wirkung ist ebenfalls außerordentlich und völlig unerwartet, insbesondere in Bezug auf die Vollständigkeit der Symptombeseitigung und das schnelle Ansprechen der Behandlung (siehe hierzu insbesondere Beipiel 4). Wenn das Amantadin entsprechend der Erfindung insbesondere während depressiver Episoden oder während akuter Krankheitsphasen anderer mit BDV-Infektionen assoziierter Störungen gegeben wird, tritt nach wenigen Tagen eine deutliche und anhaltende Besserung der Symptome auf, wie sie mit anderen Therapeutika in den seltensten Fällen beobachtet werden kann.

Die Medikamentierung mit Amantadinen entsprechend der Erfindung ist demnach im engeren Sinne indiziert bei BDV-infizierten Patienten mit Affekterkrankungen, insbesondere in der depressiven Phase, und im weiteren Sinne bei BDV-infizierten Patienten mit Störungen, die mit BDV korreliert sind, wie zum Beispiel Funktionsstörungen des limbischen Systems oder funktionelle Temporallappenstörungen, chronisches Müdigkeitssyndrom ("chronic fatigue syndrome"), Angststörungen, Zwangsstörungen und schizoaffektive Psychosen.

Nach DSM-IV ist eine Indikation vor allem bei folgenden Diagnosenummern gegeben:

Affektstörungen (Mood disorders): 296.xx (insbesondere 296.3x, 296.5x, 296.6x, 296.7x, 296.8x, 296.90); 300.3; 300.4; 311; 293.83; 295.70; Angststörungen, insbesondere in Kombination mit Depression: 300.00; 300.02; 300.21; 300.22.

Nach ICD-10 bei folgenden Diagnosenummern/Kategorien:

Affektstörungen: F3 (insbesondere F32, F33, F34, F38, F25); Angststörungen: F41; Zwangsstörungen: F42.

Die zu empfehlende Dosis liegt beim Menschen zwischen 100 und 300 mg/d (Milligramm pro Tag), vorzugsweise bei 200 mg/d, bzw. bei 0,01 bis 6 mg/kg Körpergewicht/Tag.

Die derzeit als sinnvoll angenommene Therapiedauer (kurativ) beträgt 3 bis 6 Monate.

Die gemäß der Erfindung beanspruchten Verbindungen sind auch im symptomfreien Intervall bei BDV-infizierten Patienten mit den oben bezeichneten Störungen zur Phasenprophylaxe indiziert. Eine vorbeugende Gabe von Amantadinen oder entsprechend wirkenden Stoffen ist auch bei gesunden Personen indiziert, die in engem häuslichen Kontakt mit BDV-infizierten Patienten (Mensch oder Tier) leben und/oder ein genetisches Risiko zur Entwicklung einer der oben bezeichneten Störungen haben.

Die zu empfehlende Dosis für eine prophylaktische Anwendung liegt bei bis zu 200 mg/d, vorzugsweise bei 100 mg/d für die Dauer von ca. 1 - 3 Monaten.

Als Applikationsformen kommen prinzipiell alle Formen in Betracht, die eine Resorption ermöglichen, z. B. parenteral, intramusculär, subcutan, intradermal oder topisch. Bevorzugt ist die orale Applikation in Form von Pulver, (Film-)Tabletten, Dragees, Kapseln oder ähnlichem. Bei bestimmten Anwendungen können auch transdermale Applikationsformen, insbesondere mit Depotwirkung in Betracht kommen, wie z. B. Pflaster.

Die Verträglichkeit der Amantadine ist generell gut. Amantadin wird bei oraler Gabe nahezu vollständig resorbiert. Die Ausscheidung erfolgt in unveränderter Form über die Niere. Die Halbwertszeit liegt bei ca. 15 Stunden. Bei Patienten mit Niereninsuffizienz ist vorzugsweise Rimantadin indiziert, weil es in der Leber metabolisiert wird und daher die Medikamentenclearance nicht von der Nierenfunktion abhängt.

Die pharmakologische Wirkung wird - obwohl der Mechanismus noch nicht bekannt ist - nach derzeitigem Wissensstand wesentlich der Adamantanstruktur zugeschrieben.

Zu einer Therapie und Prophylaxe im Sinne der Erfindung grundsätzlich geeignet sind daher wasserlösliche Adamantan-Derivate wie die oben genannten Adamantanamine, bzw. deren pharmakologisch vertretbare Salze, insbesondere N-(1-Adamantyl)amine und (1-Adamantyl)alkylamine bzw. deren pharmakologisch vertretbare Salze.

Die erfindungsgemäße Verwendung von Amantadinen zur Behandlung von Affekterkrankungen kann bei Menschen und bei Tieren erfolgen. Insbesondere von Pferden, Schafen, Rindern und auch Katzen ist bekannt, daß sie durch persistente Bornavirus-Infektionen erkranken können und dann auch Verhaltensstörungen, d. h., psychische Krankheitssymptome zeigen. Gerade BDV-infizierte Pferde sind je nach Belastung besonders gefährdet für die Entwicklung von Verhaltensstörungen mit Apathie als Leitsymptom und können ohne Behandlung bei vulminanter Virusproduktion tödlich erkranken.

Die Behandlung von Tieren kann auch epidemologisch angezeigt sein, um das Auftreten von BDV-Infektionen insgesamt einzudämmen und die Verbreitung zu verhindern. Da noch keine Erkenntnisse über den Übertragungsweg des Virus vorliegen, ist hierin auch eine vorbeugende Maßnahme für die Gesunderhaltung des Menschen zu sehen.

Im folgenden wird die Erfindung anhand von Beispielen illustriert.

### BEISPIELE

### Beispiel 1 (in vitro):

Junge Kaninchengehirnzellen wurden einmal mit Amantadinsulfat und in einem vergleichsversuch mit meso-Inositol behandelt.

Beide Proben wurden 1 Stunde nach dieser Behandlung mit einem humanen Borna Disease Virus-Stamm infiziert (100 +/- 10 ffu/ml mit BDV-HuH1 (zu diesem Stamm siehe auch L. Bode, et al., Mol. Psychiatry 1996, 1(3), 200 - 212)).

Amantadinsulfat hemmt die Infektion von Kaninchengehirnzellen mit humanem BDV nach dieser einstündigen Vorbehandlung zu 50 % mit einer Konzentration von nur 0,019 µg/ml. Der maximale Hemmeffekt (80 - 90 %) ist bei 0,1 µg/ml erreicht. Die Höchstdosis in vitro, die ein normales unbeeinträchtigtes Zellwachstum erlaubt, beträgt, bezogen auf das Zollsystem, 1,2 µg/ml.

Die im Patienten bei anderer Indikation (Parkinson-Syndrom) empfohlene und bezüglich der Verträglichkeit daher bereits gut erprobte Dosis von 100 - 200 mg täglich entspricht einem Spiegel von 0,2 - 0,4 µg/ml, d. h. dem Vierfachen der in vitro effektiven Dosis.

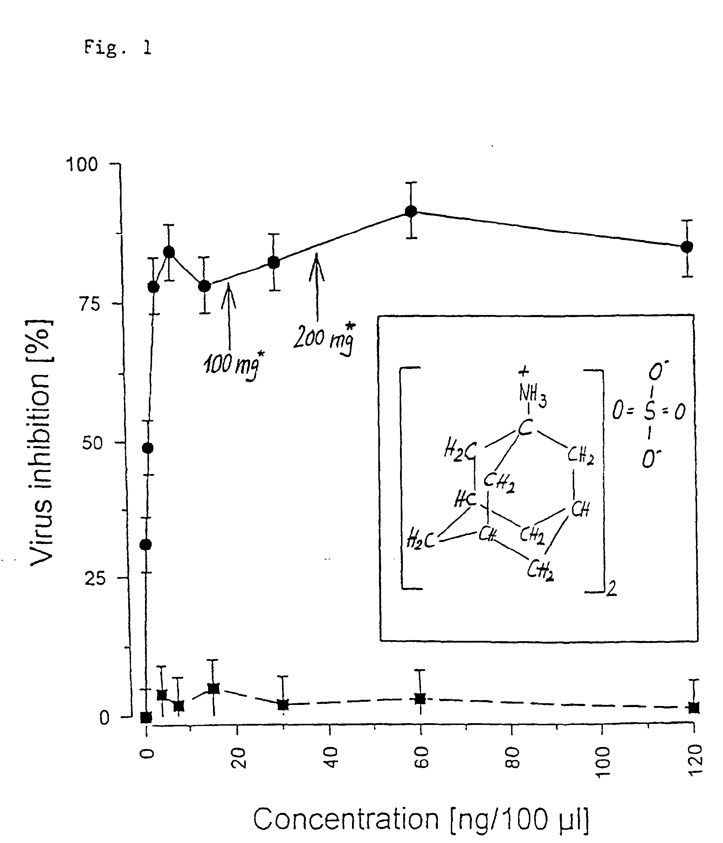

### (siehe hierzu Fig. 1)

### Beispiel 2 (in vitro):

Es wurde die Inhibierung der BDV-Replikation durch Amantadinsulfat getestet. Hierfür wurden persistent mit BDV-Hu-H1 infizierte humane oligodendrogliazellen (OL-Zellen) 6 Tage in vitro mit 1,2 µg/ml Amantadinsulfat behandelt. Auf der anderen Seite wurden frisch infizierte OL-Zellen mit der gleichen Arzneimittel-Dosis 24 Stunden vor und dann weitere 6 Tage ab der Infektion behandelt. Die Reduktion von infektiösem Virus erfolgt selbst bei bereits infizierten humanen Oligodendrogliazellen um mehr als das 10000fache. Die Reduktion der Virusmenge (Virustiter) zu nicht mehr detektierbarem BDV ist bereits nach 2 Tagen erreicht. Das Zellwachstum wurde über die ganze beobachtete Zeit mit der verwendeten Dosis von 1,2 µg/ml Amantadinsulfat praktisch nicht beeinträchtigt.

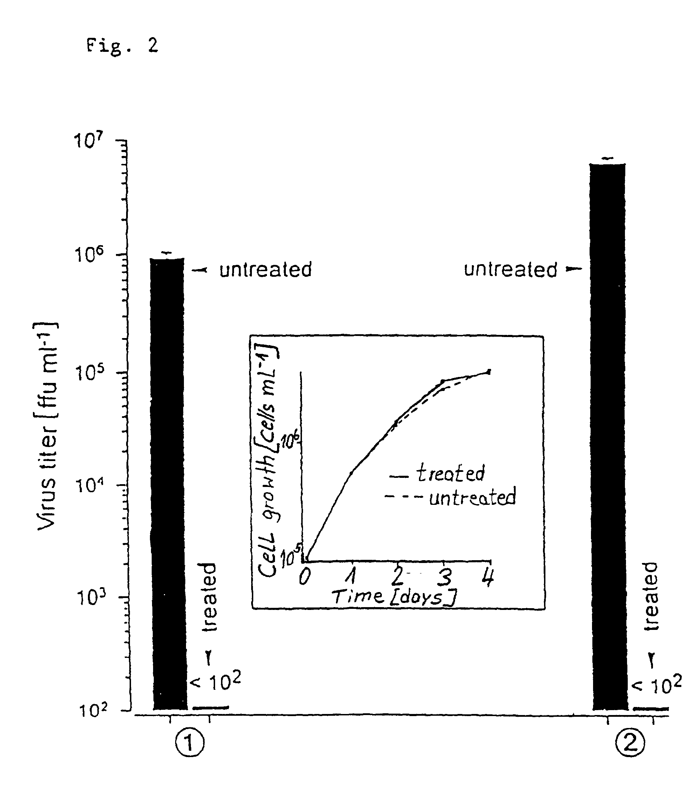

### (siehe Fig. 2)

### Beispiel 3 (in vitro):

Die Behandlung mit humanem BDV infizierter Oligodendroglia-Zellen mit 1,2 µg/ml Amantadinsulfat in vitro wurde nach 6 Tagen für die folgenden 6 Tage unterbrochen. Die Zellen wurden jeden Tag passagiert. Auch nach dem behandlungsfreien Intervall war keine BDV-Infektion mehr festzustellen. Daraus ist zu schließen, daß nicht nur die Replikation (Vermehrung) von BDV in Gegenwart von Amantadinsulfat sehr effizient verhindert wird, sondern daß Amantadine infizierte Zellen vermutlich dauerhaft von der BDV-Infektion befreien können (Virus Clearance).

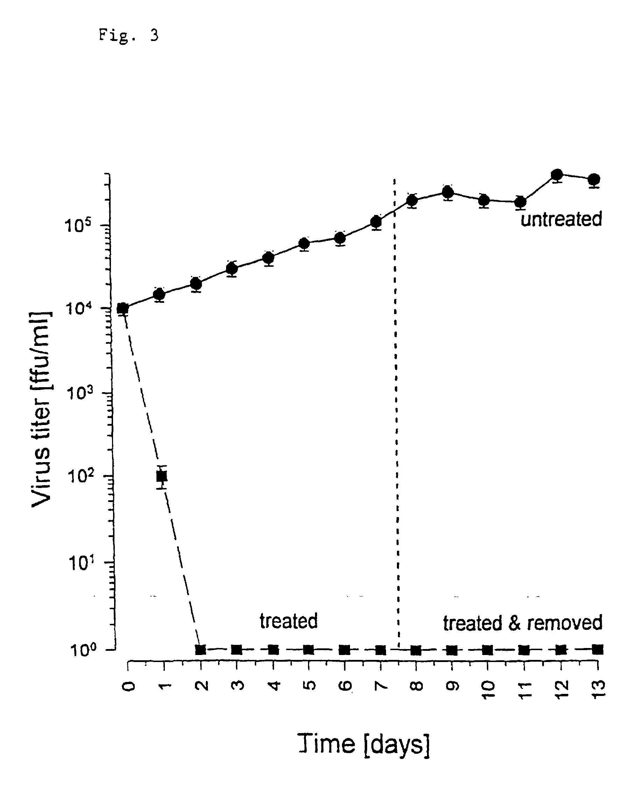

### (siehe Fig. 3)

### Beispiel 4 (in vivo):

Bei einer 67 Jahre alten Patientin mit einer seit 11 Jahren bestehenden bipolaren Erkrankung (DSM IV: 296.53) und häufigen Krankenhausaufenthalten aufgrund manischer und schwerer depressiver Episoden wurde durch Nachweis von BDV-Proteinen in Leukozyten eine aktivierte BDV-Infektion festgestellt. Die Blutuntersuchung fand drei Monate nach Beginn einer schweren depressiven Episode statt, die sich unter herkömmlicher Therapie (u.a. Mianserin und Valproat) nicht wesentlich gebessert hatte.

Die Patientin erhielt zunächst 50 - 100 mg Amantadinsulfat täglich an Tag 1 bis 3, dann 200 mg über 6 Wochen und weitere 12 Wochen 100 mg, danach weitere 2 Wochen bis auf weiteres wieder 200 mg. Ihre Depression besserte sich unter der Therapie dramatisch ab dem 8. - 11. Tag. Ab dem 15. Tag konnte sie aus dem Krankenhaus entlassen werden. Bornavirus-Aktivität (Proteinexpression in den Blutleukozyten) war ab dem 11. Behandlungstag und auch noch 5 Monate später nicht mehr nachweisbar. Die Patientin zeigte lediglich niedrige Antikörpertiter gegen BDV.

Der therapeutische Effekt war stärker und wesentlich schneller als jemals zuvor bei mit herkömmlichen Antidepressiva erzielten Besserungen. Die depressive Symptomatik ist nach psychologischen Testmethoden und nach dem subjektiven Befinden seit der dramatischen Beserung nicht mehr vorhanden. Unerwünschte Nebenwirkungen traten bisher nicht auf. Der Patientin geht es nach wie vor sehr gut.

Nach diesem Verlauf ist auch ein prophylaktischer Effekt gegen manische Phasen wahrscheinlich. Bei der Patientin folgte bis 1993 nach Besserung der Depression die manische Phase direkt, d.h. ohne krankheitsfreies Intervall. In den letzten 3 Jahren überwogen depressive Episoden mit anschließender hypomanischer Nachschwankung. Das längste symptomarme Intervall betrug 4 Monate. Unter Amantadintherapie ist die Patientin jetzt 5 Monate depressionsfrei, ohne daß eine hypomanische Nachschwankung eingetreten ist.

### Legende für die Figuren:

- **Fig. 1**: **Inhibierung einer BDV-Infektion mit Amantadinsulfat in vitro**
Behandlung von Kaninchengehirnzellen mit entweder Amantadinsulfat (durchgezogene Linie) oder meso-Inositol (unterbrochene Linie) eine Stunde vor Infektion (100 +/- 10 ffu/ml) mit einem humanen BDV-Stamm (BDV-Hu-H1)
* entsprechende dem Patienten zugeführte therapeutische Tagesdosis Amantadinsulfat in mg
- **Fig. 2**: **Inhibierung der BDV-Replikation durch Amantadinsulfat** Abnahme der Infektiösität 1) in persistent mit BDV-Hu-Hl infizierten humanen Oligodendroglia (OL-) Zellen, die 6 Tage mit 1,2 µg/ml Amantadinsulfat behandelt worden waren, und 2) in frisch infizierten OL-Zellen, die mit derselben Dosis 24 Stunden vor der Infektion und dann für weitere 6 Tage behandelt worden waren, im Vergleich zu unbehandelten Kontrollproben.
eingeblendet: Zellwachstum von nicht-infizierten OL-Zellen mit (geschlossene Linie) und ohne (unterbrochene Linie) Amantadinsulfat (1,2 µg/ml)
- **Fig. 3**: **Virus-clearance nach Unterbrechung der Behanldung mit Amantadinsulfat.**
Abnahme des Virustiters in einer frisch mit BDV-Hu-H1 infizierten humanen Oligodendroglia (OL-)Zell-Linie, die 6 Tage mit 1,2 µg/ml Amantadinsulfat behandelt worden war und anschließend 8 weitere Tage bei täglicher Passagierung (1:2) unbehandelt gehalten wurde (unterbrochene Linie), im Vergleich zu frisch infizierten, aber im gesamten Zeitraum unbehandelten OL-Zellen.

## Patentansprüche

1. Verwendung von Adamantanaminen (Amantadinen) zur Herstellung eines Medikamentes als Mittel zur Bekämpfung von Viren der Familie Bornaviridae, insbesondere von Borna Disease Virus, und zur Behandlung und Prophylaxe von mit Borna Disease Virus-Infektionen verbundenen Störungen bei Mensch und Tier, wobei das Amantadin ein 1-Amino-substituiertes, ein 1-Alkylamino-substituiertes oder ein 1-Aminalkyl-substituiertes Adamantan, bzw. ein pharmazeutisch vertretbares Salz hiervon ist.

2. Verwendung nach Anspruch 1, **dadurch gekennzeichnet, dass** die Störungen Affekterkrankungen oder kognitive cerebrale Störungen sind, oder solche Störungen, die im limbischen System verursacht sind und/oder sich ausdrücken in z.B. chronischem Müdigkeitssyndrom oder Temporallappenstörungen.

3. Verwendung nach Anspruch 2, **dadurch gekennzeichnet, dass** die Affekterkrankungen insbesondere mit einer depressiven Symptomatik kombinierte Angststörungen oder Zwangsstörungen sind.

4. Verwendung nach einem der Ansprüche 1 bis 3, wobei das Adamantanamin in Form eines pharmakologisch vertretbaren Salzes vorliegt, z.B. als Amantadinsulfat oder Amantadinhydrochlorid.

5. Verwendung nach einem der Ansprüche 1 bis 4, **dadurch gekennzeichnet, dass** das Medikament in einer Form vorliegt, die zur oralen, patenteralen, intramuskulären, subkutanen, intradermalen oder topischen Verabreichung formuliert ist.

6. Verwendung nach einem der Ansprüche 1 bis 5, **dadurch gekennzeichnet, dass** das Medikament in einer Form vorliegt, die zur Gabe in einer Dosis von >0 bis 10 mg/kg Körpergewicht pro Tag, vorzugsweise 1 - 4 mg/kg Körpergewicht pro Tag formuliert ist.

## Claims

1. Use of adamantanamines (amantadines) for producing a medicament as means for controlling viruses of the Bornaviridae family, especially of Borna disease virus, and for the treatment and prophylaxis of disorders associated with Borna disease virus infections in humans and animals, where the amantadine is a 1-amino-substitued, a 1-alkylamino-subsituted or a 1-aminoalkyl-substituted adamantane, or a pharmaceutically acceptable salt thereof.

2. Use according to claim 1, **characterized in that** the disorders are affective disorders or cognitive cerebral disorders, or disorders which are caused in the limbic system and/or manifest themselves by, for example, chronic fatigue syndrome or temporal lobe disorders.

3. Use according to claim 2, **characterized in that** the affective disorders are anxiety disorders or obsessive-compulsive disorders combined in particular with depressive symptoms.

4. Use according to any of claim 1 to 3, where the adamantanamine is in the form of a pharmacologically acceptable salt, e.g. as amantadine sulphate or amantadine hydrochloride.

5. Use according to any of claim 1 to 4, **characterized in that** the medicament is in a form which is formulated for oral, parenteral, intramuscular, subcutaneous, intradermal or topical administration.

6. Use according to any of claims 1 to 5, **characterized in that** the medicament is in a form which is formulated for administration in a dose of > 0 to 10 mg/kg of bodyweight per day, preferably 1-4 mg/kg of bodyweight per day.

## Revendications

1. Utilisation d'amines d'adamantane (Amantadines) pour la préparation d'un médicament en tant que remède pour lutter contre les virus de la famille des Bornaviridae, en particulier les virus de la maladie de Borna et pour le traitement et la prophylaxie d'affections liées aux infections par le virus de la maladie de Borna, chez l'homme et l'animal, dans laquelle l'amantadine est une adamantane 1-Amino-substituée, une 1-Alkylamino-substituée ou une 1-Aminalkyl-substituée, en particulier un sel pharmaceutiquement acceptable de celles-ci.

2. Utilisation selon la revendication 1, **caractérisée en ce que** les affections sont des maladies affectives ou des affections cérébrales cognitives ou des affections analogues, qui affectent le système limbaire et / ou qui s'expriment par exemple par un syndrome de fatigue chronique ou par des affections des lobes temporaux.

3. Utilisation selon la revendication 2, **caractérisée en ce que** les maladies affectives sont en particulier des affections dues à l'anxiété ou à la contrainte combinées avec une symptomatique dépressive.

4. Utilisation selon l'une des revendications 1 à 3, dans laquelle l'amine d'adamantane est sous la forme d'un sel pharmaceutiquement acceptable tel qu'un sulfate d'adamantane ou un chlorhydrate d'adamantane.

5. Utilisation selon l'une des revendications 1 à 4, **caractérisée en ce que** le médicament est présenté sous une forme formulée pour l'administration orale, parentérale, intramusculaire, sous-cutanée, intradermique ou topique.

6. Utilisation selon l'une des revendications 1 à 5, **caractérisée en ce que** le médicament est présenté sous une forme formulée pour l'administration d'une dose supérieure à 0 jusqu'à 10 mg/Kg de poids corporel par jour, de préférence 1-4 mg/Kg de poids corporel par jour.
